# EUROPÄISCHE PATENTANMELDUNG

(11) **EP 1 661 531 A1**
(43) Veröffentlichungstag der Anmeldung: **31.05.2006**
(21) Anmeldenummer: 05023087.9
(22) Anmeldetag: 22.10.2005
(51) Int. Cl.: A61F 2/44, A61F 2/30

(54) **Implantat für die Endoprothetik**

(30) Priorität: 30.11.2004 DE 102004057977
(71) Anmelder: MTM Medizintechnik Mauk GmbH, 22844 Norderstedt (DE)
(72) Erfinder: Mauk, Rudolf, 22844 Norderstedt (DE)
(74) Vertreter: Hansmann, Dierk

(57) **Zusammenfassung**

Bei einem in eine Körperhöhle einsetzbaren Implantat mit verstellbaren außenliegenden Begrenzungen ist vorgesehen, daß in einem Grundkörper (15) verstellbare Kolbenelemente (10) angeordnet sind. Die Kolbenelemente werden durch einen Kraftumlenk-Mechanismus (1) verstellt, der durch einen im Grundkörper abgewinkelten Kanal (2) mit aufgenommenen aneinanderstoßenden Druckkörpern (3) gebildet ist. Hierbei werden die Druckkörper in einem Endbereich gegen das Kolbenelement gepreßt und die Stellbewegung über eine Schraube (8) als verschlußelement vom anderen Ende eingeleitet.

## Beschreibung

Die Erfindung bezieht sich auf ein Implantat für die Endoprothetik, wie zur Ausbildung von Wirbelkörperersatzelementen, für einen Einsatz in Körperhöhlen mit verstellbaren außenliegenden Begrenzungen zum Spreizen und Fixieren benachbarter Körperteile mit einer Verstellung über einsetzbare Werkzeuge, wobei die außenliegenden Begrenzungen als verstellbare Elemente in einem Grundkörper gebildet sind.

Bei Implantaten dieser Art ist es bekannt, eine Höhenregulierung durch Gewinderinge oder durch Zahnstangen durchzuführen. Da die Einsatzorte oftmals wegen eines kleinen Operationsfeldes schwer erreichbar sind, ist eine Einstellung umständlich oder es müssen Reibekräfte überwunden werden, die bereits beim Spreizvorgang zum Verschleiß der Bauteile führen. Normalerweise ist bei diesen bekannten Systemen auch ein zusätzlicher Arbeitsgang zur Sicherung der eingestellten Höhen, wie durch Schrauben, erforderlich.

Es besteht bei gattungsgemäßen Implantaten zusätzlich die Anforderung, daß aus anatomischen Gründen eine kleine Dimensionierung vorliegen muß und somit auch der Mechanismus zur Erzielung der notwendigen großen Kräfte für die Verspannung auf kleinem Raum zu realisieren sind.

Es ist ferner zu berücksichtigen, daß nur wenige zugelassene Werkstoffe für die vorliegende empfindliche biologische Umgebung zur Ausbildung der Implantate zur Verfügung stehen und ein Mechanismus nur aus wenigen Teilen bestehen darf, die verschleißfrei funktionieren und durch einen Sterilisationsvorgang keinen Schaden nehmen.

Die Aufgabe der Erfindung ist es, eine Anwendung der gattungsgemäßen Art zu verbessern, die auch einfache Weise eine relativ kleine Bauweise gewährleistet und eine im Winkel zur Verstellachse angeordnete Einstellung ermöglicht.

Die Lösung dieser Aufgabe erfolgt erfindungsgemäß dadurch, daß im Grundkörper ein Kanal ausgebildet ist, der einerseits durch ein im Grundkörper geführtes Kolbenelement abgeschlossen und andererseits der Kanal im Winkel zur Verstellachse nach außen zu einer Öffnung geführt ist, wobei der Kanal eine Vielzahl von aneinanderstoßende Druckkörper aufnimmt und der Öffnung ein verstellbares Verschlußelement zur Verschiebung der Druckkörper aufweist.

Hierdurch ist es möglich, die Umlenkung einer Einstellbewegung auf einfache Weise durchzuführen und eine Verspannung durch eine beispielsweise seitliche Einstellung vorzunehmen.

Ferner wird vorgeschlagen, daß beide außenliegende Begrenzungen des Grundkörpers durch Kolbenelemente gebildet sind und der Kanal zur Aufnahme der Druckkörper eine Verzweigung zur Verbindung mit Kolbenelementen auf beiden Seiten aufweist.

Eine einfache Ausbildung mit einer guten Funktionsfähigkeit wird dadurch geschaffen, daß die Druckkörper als Kugeln oder Zylinderelemente ausgebildet sind.

Zur Einstellung einer Höhenverstellung ist vorgesehen, daß das verschlußelement als verstellbarer Gewindestift mit einem korrespondierenden Gewinde ausgebildet ist.

Um eine Auffüllung des Implantates mit Knochengewebe zu ermöglichen, ist vorgesehen, daß der Grundkörper als Kolbenelement mit Aufnahmeräumen und Öffnungen zum Außenbereich ausgebildet ist.

Um eine stabile Zuordnung des eingesetzten Implantates zu ermöglichen, ist vorgesehen, daß die Kolbenelemente im Grundkörper durch korrespondierende Führungselemente verdrehsicher angeordnet sind.

Zur Anpassung an den Einsatzort wird vorgeschlagen, daß die außenliegende Begrenzung des Kolbenelementes durch austauschbare unterschiedliche Endplatten gebildet ist.

Weiterhin ist vorgesehen, daß die Endplatten Dorne zur Verkrallung im angrenzenden Bereich aufweisen.

In den Zeichnungen sind Ausführungsbeispiele für ein Wirbelkörperersatzelement schematisch dargestellt. Es zeigen:
- Fig. 1: eine Schnittdarstellung eines Implantates in seiner Ausgangsposition;
- Fig. 2: eine Darstellung wie Fig. 1 in der verstellten Spreizposition;
- Fig. 3: eine Ausschnittdarstellung für die Verstellung über einen verzweigten Kanal mit beidseitigen Kolbenelementen im Grundkörper und
- Fig. 4: eine Darstellung wie Fig. 1 mit modularen Ergänzungselementen als Endplatten.

Bei den gezeigten Anordnungen ist ein Grundkörper 15 als Wirbelkörperersatzelement angeordnet, der ein geführtes Kolbenelement 10 aufweist. Im Grundkörper 15 ist ein Kanal 2 ausgebildet, der im Winkel verläuft. Der Kanal 2 nimmt eine Vielzahl von aneinanderstoßenden Druckkörper 3 auf, die in dieser Ausbildung als Kugeln ausgebildet sind und mit dem Kanal 2 ein Kraftumlenk-Mechanismus 1 bilden. Hierzu ist der Kanal 2 einerseits durch das Kolbenelement 10 abgeschlossen und am anderen Ende über eine Öffnung 7 durch eine Schraube 8 als Gewindestift verschlossen, die über ein zugeordnetes Innengewinde 6 des Kanals 2 eingreift und über einen Innensechskant 14 mittels eines Werkzeuges von außen zur Einstellung der Hubbewegungen des Kolbenelementes 10 über seinen Boden 11 verstellbar.

Der Kanal 2 besitzt hierbei eine Krümmung 4, wobei die Kugeln 3 die Krümmung 4 passieren können. Diese Krümmung kann in verschiedenen Winkeln ausgelegt sein, wobei in diesem Fall eine Schräge 5 angeordnet ist.

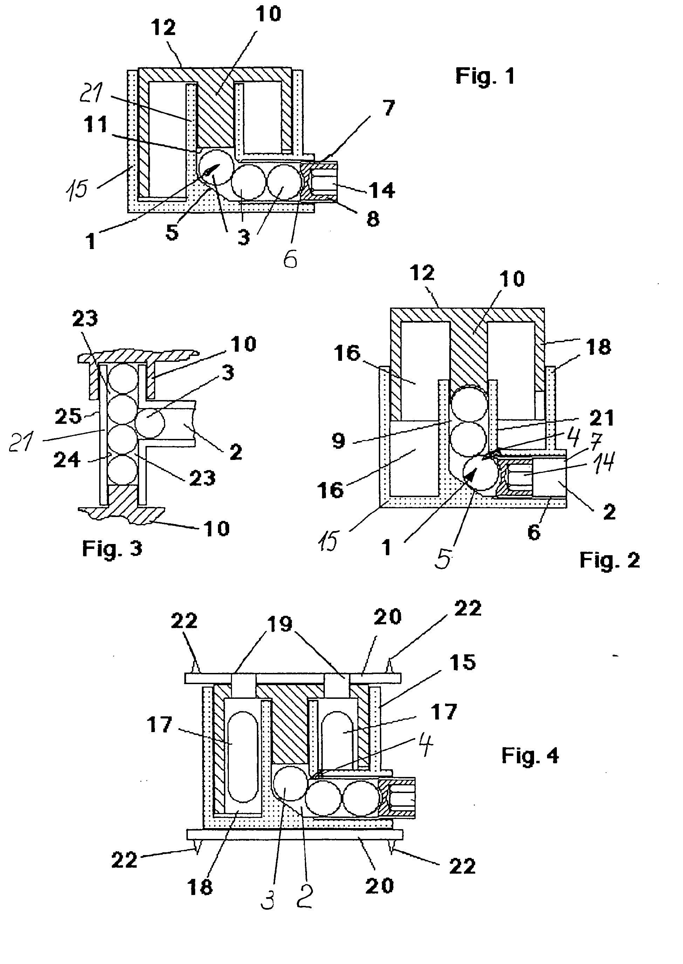

Es kann der Kanal 2 auch, wie in Fig. 3 dargestellt, eine Gabelung in zwei Seitenarme 23 aufweisen, die zur Verschiebung der Kugeln 3 gestaltet sind und auf jeder Seite ein Kolbenelement 10 angeordnet ist.

Das Kolbenelement ist generell nicht unbedingt durch die Innenseite 24 der Wandung 21 geführt. Es ist auch vorstellbar, daß ein Kolbenelement 10 die Außenseite 25 einer Wandung 21 geführt wird und der Boden 11 nicht in den Bereich 9 des Kanals 2 oder in einen Seitenarm 23 hineinragt.

Das Kolbenelement 10 ist als Bauteil 12 gegebenenfalls so ausgelegt, daß es sich als Spreizelement des Wirbelkörperersatzelementes 15 eignet.

Wird die Schraube 8 in den Kanal 2 mit den aufgenommenen Kugeln 3 eingeschraubt, werden die Kugeln 3 im Kanal 2 der Krümmung 4 und dem anschließenden Bereich 9 des Kanals 2 verschoben und drücken gegen das Kolbenelement 10. Die Stellbewegung wird durch die Eindrehtiefe der Schraube 7 bestimmt. Auch kann ein Anschlag, der das Herausdrücken des Kolbenelementes 10 begrenzt, vorgesehen werden.

Um zu verhindern, daß die Kugeln 3 beim Herausdrehen der Schraube 7 ungewollt den Kanal 2 verlassen, ist ein Sicherungsmechanismus im Kanal 2 anzubringen. Entsprechend der Anwendung des Kanals 2, der Winkel der Krümmung 4, die Größe der Schraube 8 und die Anzahl und Form der Körper 3 festgelegt.

Als Spreizelement eines Wirbelkörperersatzelementes 15 ist es möglich, einen relativ großen Raum 16 innerhalb des Grundkörpers 15 zu bilden. Dieser Raum 16 kann mit Knochengewebe ausgefüllt werden. Dies kann durch Öffnungen 17 in der Wandung 18 erfolgen. Durch die Öffnung 17 in der Wandung 18 und durch Öffnungen 19 in zugeordnete Endplatten 20 des Grundkörpers kann das Knochengewebe hindurchwachsen.

Der Grundköper ist modular aufgebaut. Am außenliegenden Bereich 12 des Hubelementes 10 sind unterschiedliche Endplatten 20 aufsetzbar, wobei eine Anpassung an unterschiedliche anatomische Verhältnisse möglich ist. Die Endplatten 20 besitzen Dorne 22, die für eine feste Verkrallung mit angrenzenden Wirbelkörpern sorgen. Auch sind die Endplatten 20 gegebenenfalls mit einer Beschichtung versehen, die eine knöcherne Integration begünstigt. Anstatt einer Endplatte 20 ist auch eine Bandscheibenprothese aufsetzbar.

## Patentansprüche

1. Implantat für die Endoprothetik, wie zur Ausbildung von Wirbelkörperersatzelementen, für einen Einsatz in Körperhöhlen mit verstellbaren außenliegenden Begrenzungen zum Spreizen und Fixieren benachbarter Körperteile mit einer Verstellung über einsetzbare Werkzeuge, wobei die außenliegenden Begrenzungen als verstellbare Elemente in einem Grundkörper gebildet sind, **dadurch gekennzeichnet, daß** im Grundkörper (15) ein Kanal (2) ausgebildet ist, der einerseits durch ein im Grundkörper (15) geführtes Kolbenelement (10) abgeschlossen und andererseits der Kanal (2) im Winkel zur Verstellachse nach außen zu einer Öffnung (7) geführt ist, wobei der Kanal (2) eine Vielzahl von aneinanderstoßende Druckkörper (3) aufnimmt und der Öffnung (7) ein verstellbares Verschlußelement (8) zur Verschiebung der Druckkörper (3) aufweist.

2. Implantat nach Anspruch 1, **dadurch gekennzeichnet, daß** beide außenliegende Begrenzungen (12) des Grundkörpers (15) durch Kolbenelemente (10) gebildet sind und der Kanal (2) zur Aufnahme der Druckkörper (3) eine Verzweigung (23) zur Verbindung mit Kolbenelementen (10) auf beiden Seiten aufweist.

3. Implantat nach Anspruch 1 oder 2, **dadurch gekennzeichnet, daß** die Druckkörper (3) als Kugeln oder Zylinderelemente ausgebildet sind.

4. Implantat nach einem der Ansprüche 1 bis 3, **dadurch gekennzeichnet, daß** das Verschlußelement (8) als verstellbarer Gewindestift mit einem korrespondierenden Gewinde (7) ausgebildet ist.

5. Implantat nach einem der Ansprüche 1 bis 4, **dadurch gekennzeichnet, daß** der Grundkörper (15) als Kolbenelement mit Aufnahmeräumen (16) und Öffnungen (17) zum Außenbereich ausgebildet ist.

6. Implantat nach einem der Ansprüche 1 bis 5, **dadurch gekennzeichnet, daß** die Kolbenelemente (10) im Grundkörper (15) durch korrespondierende Führungselemente verdrehsicher angeordnet sind.

7. Implantat nach einem der Ansprüche 1 bis 6, **dadurch gekennzeichnet, daß** die außenliegende Begrenzung (20) des Kolbenelementes (10) durch austauschbare unterschiedliche Endplatten (20) gebildet ist.

8. Implantat nach einem der Ansprüche 1 bis 7, **dadurch gekennzeichnet, daß** die Endplatten Dorne (22) zur Verkrallung im angrenzenden Bereich aufweisen.
